Europäisches Patentamt

European Patent Office

Office européen des brevets

(11) Publication number: **0 375 151**

**A1**

## (12) EUROPEAN PATENT APPLICATION

(21) Application number: **89311877.8**

(22) Date of filing: **16.11.89**

(51) Int. Cl.5: **A61F 11/00**

(30) Priority: **19.12.88 US 286744**

(43) Date of publication of application:
**27.06.90 Bulletin 90/26**

(84) Designated Contracting States:
**DE FR GB IT**

(71) Applicant: **MINNESOTA MINING AND MANUFACTURING COMPANY**
**3M Center, P.O. Box 33427**
**Saint Paul, MN 55133- 3427(US)**

(72) Inventor: **Stypulkowski, Paul H. c/o Minnesota Mining and**
**Manufacturing Co. 2501 Hudson Road P.O. Box 33427**
**St. Paul Minnesota 55133-3427(US)**

(74) Representative: **Baillie, Iain Cameron et al**
**c/o Ladas & Parry Isartorplatz 5**
**D-8000 München 2(DE)**

(54) **Apparatus and method for suppressing tinnitus.**

(57) Method and apparatus for suppressing tinnitus by repositioning the basilar membrane so that the tectorial membrane does not chronically contact the stereocilia of the inner row of hair cells. A preferred mechanism involves the application of a pressure differential to the external ear canal. Preferably, the pressure differential is negative with respect to ambient. Alternatively, a positive pressure may be maintained in the external ear canal and, preferably, statically maintained. Alternative arrangements for repositioning the basilar membrane involve repositioning the ossicles and providing a middle ear pressure either with or without venting to the external ear canal.

FIG. 7

Xerox Copy Centre

## APPARATUS AND METHOD FOR SUPPRESSING TINNITUS

### Technical Field

The present invention relates generally to auditory methods and prostheses and more particulary to auditory methods and prostheses for the suppression or elimination of tinnitus.

### Background Art

The problem of tinnitus is well known. Many individuals have suffered and currently do suffer from tinnitus. Tinnitus, simply defined, is the perception of sound in the ear which does not, in fact, exist, i. e., is not present in the external environment. These sounds typically may be characterized as a ringing, whistling, roaring or buzzing. Tinnitus has been reported to affect up to thirty percent (30%) of the United States population. Individuals with tinnitus may have symptoms ranging from mild to severe and ranging from chronic to intermittent. Several million people in the United States suffer debilitating tinnitus which interferes with everyday activities. In some cases, tinnitus can be chronic and severe, debilitating the individual.

Several attempts have been made in the past to mask or "cure" tinnitus. Some of these attempts have involved treating the tinnitus by the administration of drugs. An example of these attempts is disclosed in U. S. Patent No. 4,735,968, Guth.

In some cases the tinnitus is so severe that drastic steps have been taken to eliminate the sensation. The symptom of tinnitus can sometimes be "cured" by a section of the VIIIth nerve in one ear, completely eliminating any sensation of hearing in that ear. An example of a drastic attempt to "cure" tinnitus is disclosed in U. S. Patent No. 4,408,602, Nakajima.

Some prior attempts for suppressing tinnitus have involved the use of electrical stimulation.

An example of this type of electrical stimulation is described in the ATA Newsletter, Vol. 10, No. 1, American Tinnitus Association, pp. 1-4 (March 1985). Electrical stimulation, however does not actually suppress tinnitus but rather simply attempts to mask the existing tinnitus sound with another sound created by the electrical stimulus signal. The ultimate goal of electrical stimulation is to suppress tinnitus without adding any other sounds. In some cases this is possible with direct current (DC) electrical stimulation. However, direct electrical currents cause damage to biological tissue and thus, this method cannot be practiced chronically. In some other cases, the electrical stimulation adds, rather than subtracts, from the sound environment, but for some individuals this may be more pleasant than doing nothing since the tinnitus sound may be so disturbing.

A similar example of masking, in this case With acoustic stimulation is shown in U. S. Patent No. 4,222,393, Hocks et al, Tinnitus Masker, which discloses a system in which a tinnitus patient is subjected to external sounds of differing pitches to determine the frequency of the perceived tinnitus sound and then provided with signals both above and below the determined frequency in order to mask the tinnitus. These systems do not eliminate or cure the tinnitus but merely superimpose another stimulus on top of the existing tinnitus sensation in an attempt to mask the perceived sensation.

In an article by B. Densert and O. Densert, "Overpressure in Treatment of Meniere's Disease", 92 Larynogoscope pp. 1285-1292 (November 1972), the Denserts described tests they conducted on patients suffering from Meniere's disease. It is to be pointed out that Meniere's Disease, sometimes referred to as cochlear hydrops, is a significantly different condition from tinnitus which accompanies a typical high frequency hearing loss. That is, Meniere's Disease tinnitus represents only a small portion of the tinnitus which exists in the general population. In some of the tests, they conducted local overpressure tests on the middle ear using a tube inserted through the tympanic membrane. In their tests they used a pressure increase upon which a low frequency sine wave was imposed. They require the sine wave oscillating pressure. The Denserts' main intention appears to be to treat patients with Meniere's disease. Since the main symptoms of Meniere's disease are hearing deficiency and vertigo, these are the symptoms which the Denserts have tested. In the question and answer period at the end of the presentation which resulted in this paper, in response to a question, they did say that they had had success in treating the tinnitus symptom.

Densert et al, in PCT International Published Patent Application No. WO 83/02556, An Apparatus for Influencing the Hydrodynamic System in the Inner of an Ear, describes an apparatus for treating Meniere's disease, which has primary symptoms of hearing deficiency and vertigo, with tinnitus being a secondary symptom. The apparatus is a large, stationary, office apparatus which, at certain treatment intervals, attempts to reduce the volume of the endolymph by introducing a fluctuating pressure upon the perilymphatic fluid. It is fundamental in the design of the apparatus that a patient would go to an office, be temporarily connected to the apparatus, undergo treatment and leave the office with the expectation that the changes in the endolymph would remain for a significant period of time. The pressures induced upon the perilymphatic fluid, in order to force a reduction in the volume of the endolymph, requires generating a static positive pressure level (during the treatment time period only) and generating a varying pressure level on top of the static positive pressure. The frequency of the varying pressure is described to be between 3 and 6 Hertz. Thus, the apparatus described in Densert et al PCT attempts to reduce the volume of the endolymph by temporarily inducing a fluctuating positive pressure on the endolymph in order to reduce the symptoms of Meniere's disease which have primary symptoms of hearing loss and vertigo and a secondary symptom of tinnitus.

European Patent Publication No. 0 266 474 Al, of Application No. 8650390.5, Procedure and Equipment for Treatment of Meniere's Disease, also by the Denserts, describes a very similar device. The apparatus requires the generation and transmission of complex pressure pulses in the fluid system of the inner ear in order to reduce the volume of the endolymph. As in the previous publications from the Denserts, a fluctuating, varying, or complex positive pressure is introduced to the inner ear in order to force fluid from the endolymph for the treatment of Meniere's disease, i. e., cochlear hydrops.

Disclosure of Invention

The present invention is based upon the discovery that tinnitus is caused, at least in part, by a malfunction in the cochlea.

As will be fully described later, in an individual with normal hearing, the stereocilia of the inner row of hair cells, the cells which are "connected" to the VIIIth nerve, and which transmit auditory information to the brain, are separated from the tectorial membrane by a fluid space. The three rows of outer hair cells, whose stereocilia are directly attached to the tectorial membrane, perform the function of transducing the mechanical energy of the oscillations of the fluid in the cochlea into electrical energy and reverse transducing the electrical energy generated back into mechanical energy to provide amplification and to sharpen the tuning of the system. In addition to these functions typically ascribed to them, the rows of outer hair cells, with their associated stereocilia, also function to maintain the positioning of the tectorial membrane relative to the stereocilia of the inner hair cells. The inner row of hair cells, which are strictly passive, respond to the amplified movements of the basilar membrane and/or the tectorial membrane through deflections of their stereocilia by the fluid movement between the basilar and tectorial membranes. When the inner hair cell stereocilia are deflected the inner hair cells depolarize causing them to release the neurotransmitter which excites the VIIIth nerve fibers and produces the sensation of hearing.

In cochleae with impaired hearing, particularly those damaged as a result of excessive noise, the damaged and sometimes missing stereocilia and/or three rows of outer hair cells may allow the tectorial membrane, at least along a portion of the cochlea, to chronically contact and deflect the stereocilia of the inner row of hair cells causing the hair cells to depolarize and release the neurotransmitter, thus exciting the VIIIth nerve resulting in the sensation of hearing when no sound is actually present, i. e., tinnitus.

The cure, then, for the prevention or reduction in this abnormal sensation of hearing, i.e., tinnitus, and to alter other aspects of the auditory sensation, is to separate the tectorial membrane from the stereocilia of the inner row of hair cells where contact has been made. It has been found that displacing the basilar membrane, especially a static displacement of the basilar membrane in a direction toward the scala tympani and away from the scala media, provides the desired separation and the reduction or elimination of tinnitus.

The present invention provides an apparatus for suppressing tinnitus in an auditory system having a cochlea with a basilar membrane separating the scala media from the scala tympani, the basilar membrane being normally positioned in a first position, the apparatus having a device displacing the basilar membrane from the first position to a second position suppressing the tinnitus. It is preferred that the second position, compared with the first position, is away from the scala media and toward the scala tympani. In one embodiment the displacement is a static displacement. It is preferred that the second position be selected so that the tectorial membrane is separated from the stereocilia of the inner row of hair cells. It is preferred that the basilar membrane be displaced to the second position at least for the location along the basilar membrane within the cochlea which corresponds to the location along the basilar membrane in the cochlea causing the tinnitus and that the location along the basilar membrane corresponds to the perceived frequency of the tinnitus.

The present invention also provides an improved auditory system suppressing tinnitus having a cochlea having a basilar membrane separating the scala media from the scala tympani, the basilar membrane being normally positioned in a first position; and a displacement mechanism operatively coupled to the cochlea for displacing the basilar membrane from the first position to a second position suppressing the tinnitus.

The present invention also provides an apparatus for suppressing tinnitus having an auditory system with a tympanic membrane, a middle ear and an external ear canal, the tympanic membrane being normally positioned in a first position, the apparatus having a device adapted to act upon the tympanic membrane displacing the tympanic membrane from the first position to a second position suppressing the tinnitus. In a preferred embodiment the device operates by creating a negative static pressure differential, relative to ambient, in the external ear canal.

The present invention also provides an apparatus for suppressing tinnitus having an auditory system with an ossicular chain in a middle ear and an external ear canal, the ossicular chain being normally positioned in a first position, the apparatus having a device adapted to act upon the ossicular chain displacing the ossicular chain from the first position to a second position suppressing the tinnitus.

The present invention also provides an apparatus for suppressing tinnitus having an auditory system with an ossicular chain in a middle ear and an external ear canal, the ossicular chain being normally positioned in a first position, the apparatus having a device adapted to act upon the ossicular chain displacing the ossicular chain from the first position to a second position by creating a negative, relative to ambient, pressure differential in the middle ear suppressing the tinnitus.

The present invention also provides an apparatus for suppressing tinnitus in an auditory system having a cochlea with a basilar membrane separating the scala media from the scala tympani, the basilar membrane normally physically positioned in a first position, the apparatus having a mounting mechanism adapted to be utilized in conjunction with the auditory system for mounting the apparatus to the person; and a displacement mechanism coupled to the mounting mechanism and adapted to act upon the basilar membrane for displacing the basilar membrane from the first position to a second position suppressing the tinnitus.

The present invention also provides an apparatus for suppressing tinnitus in a person having an external ear canal, having a sealing mechanism adapted to be coupled to the external ear canal for providing a substantially hermetic seal, relative to ambient, for the external ear canal; and a pressure mechanism adapted to be coupled to the external ear canal for providing a pressure, relative to ambient, in the external ear canal suppressing the tinnitus.

The present invention also provides a tinnitus suppression device adapted to be utilized in conjunction with a person having an auditory system with an external ear canal, the device having an ear plug adapted to be placed in the external ear canal of the person, the ear plug having an orifice allowing communication of fluids to and from the external ear canal with the ear plug in place within the external ear canal and a pressure mechanism coupled to the valve mechanism for providing a selected pressure to be applied to the external ear canal with the ear plug in place. Preferably, a valve mechanism is coupled to the ear plug for selectively allowing fluids to be transferred into or out of the external ear canal through the orifice.

The present invention also provides a tinnitus suppression device adapted to be utilized in conjunction with a person having an auditory system with an external ear canal, the device having a body, sized and shaped for easy insertion into the external ear canal of the person, the body having an orifice allowing for the communication of fluids in and out of the external ear canal with the device in place within the external ear canal, a sealing mechanism coupled to the body for sealing for the external ear canal from ambient and a pressure mechanism coupled to a valve providing a selected pressure to be applied to the external ear canal with the device in place. Preferably, the valve is coupled within the orifice of the body selectively allowing fluids to be transferred into or out of the external ear canal through the orifice.

The present invention also provides a tinnitus suppression device adapted to be utilized in conjunction with a person having an auditory system with an external ear canal, the device having a sealing mechanism, sized and shaped for easy insertion into the external ear canal of the person, the sealing mechanism having an orifice allowing for the communication of fluids in and out of the external ear canal with the device in place within the external ear canal for sealing the external ear canal from ambient a pressure mechanism coupled to a valve providing a selected pressure to be applied to the external ear canal with the device in place. Preferably, the valve is coupled to the sealing mechanism selectively allowing fluids to be transferred into or out of the external ear canal through the orifice.

The present invention also provides a method for suppressing tinnitus in a person having a cochlea with a basilar membrane separating the scala media from the scala tympani, the basilar membrane being normally positioned in a first position, utilizing the step of displacing the basilar membrane from the first position to a second position until the tinnitus is suppressed.

The present invention also provides a method of suppressing tinnitus in an auditory system having an external ear canal, utilizing the steps of providing a substantially hermetic seal to the external ear canal, applying a plurality of static pressures, having differing values, to the external ear canal, determining which value or values of the static pressures suppresses the tinnitus and maintaining at least one of the values of the static pressures for therapeutic benefit, whereby the tinnitus is suppressed.

The Densert publications can be distinguished from the present invention in at least four significant ways. First, the Denserts require pressures which are both positive and have a sine wave oscillating component. In direct contrast the present invention teaches, ano requires, either the application of negative pressure or the application of a static pressure to the auditory system of the patient. This treatment the Denserts do not appreciate and do not teach. The treatment in the Densert publication is limited to positive fluctuating pressures. In fact, the later European Patent Application specifies that the treatment apparatus shall be provided with a safety device rendering a negative pressure pulse impossible (see the last. sentence of page 2). Second, the Denserts do not realize, nor suggest, that the treatment they envision can be achieved by creating a pressure change in the external ear canal since all of their reported tests in the cited documents administer the fluctuating positive pressure to the middle ear space. Third, the Denserts clearly contemplate that treatment from their device can be achieved only from extensive treatment. See, for example, the later European Patent Application which states that clinical improvement could be observed only after a couple of days following several hours of treatment (see middle of page 7). Fourth, the apparatus described by the Denserts is large and bulky requiring the patients to receive treatment in the office and then leave without further treatment (until, of course they return) relying upon residual therapeutic benefit. In direct contrast, the apparatus of the present invention allows a small, portable chronically worn body aid. Thus, it is clear that the Denserts have failed to perform the treatment fundamental to the present invention, fail to utilize the apparatus of the present invention and fail to recognize and appreciate the important aspects of the present invention.

## Brief Description of Drawings

The foregoing advantages, construction and operation of the present invention will become more readily apparent from the following description and accompanying drawings in which:

Figure 1 is a cross-sectional view of the human ear;

Figure 2 is a cross-sectional view of the cochlea;

Figure 3 is an expanded cross-sectional view of a portion of the cochlea;

Figure 4 is a cross-sectional view of a portion of a normally functioning cochlea;

Figure 5 is a cross-sectional view of a portion of a cochlea which has damaged stereocilia of the three rows of outer hair cells;

Figure 6 is a cross-sectional view of the portion of the cochlea of Figure 5 with a displaced basilar membrane as taught by the present invention;

Figure 7 is a cross-sectional view of a preferred embodiment of the apparatus of the present invention in place in the external ear canal of a patient;

Figure 8 is an enlarged sectional view of a preferred embodiment of the apparatus of the present invention;

Figure 9 is a cross-sectional view of the apparatus of Figure 8;

Figure 10 is a chart of the exemplary pressures which can be used in conjunction with the apparatus and method of the present invention;

Figure 11 is a view of the apparatus of Figure 8 connected to a pressure regulating device;

Figure 12 is a view of a preferred embodiment of a pressure regulating device for use with the apparatus or method of the present invention;

Figure 13 illustrates an alternative embodiment of the apparatus of the present invention operating by action on the ossicles in place in the middle ear of patient;

Figure 14 is a more detailed view of the apparatus of Figure 13; and

Figure 15 is a cross-sectional view of an embodiment of the apparatus of the present invention operating by pressure in the middle ear space in place in the middle ear of patient;

## Detailed Description

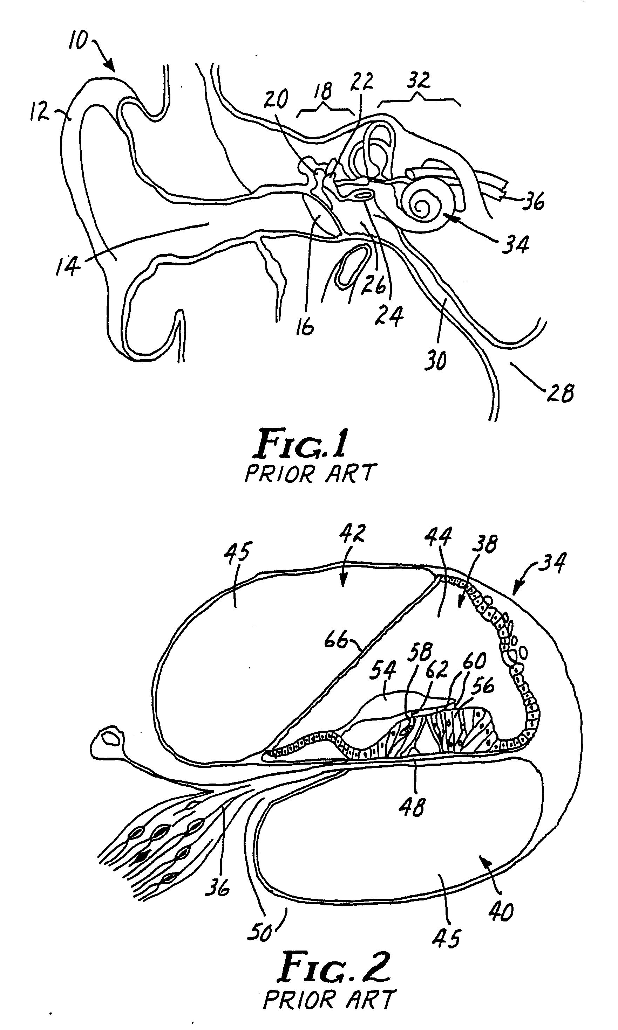

Figure 1 illustrates a cross-sectional view of the human ear 10 showing the pinna (auricle) 12, the external ear canal (external meatus) 14 and the tympanic membrane 16 (commonly called the ear drum). Auditory sounds in the environment enter the external ear canal 14 and acoustically vibrate the tympanic membrane 16. Ossicles 18 (also called the ossicular chain, and more particularly the malleus 20, the incus 22 and the stapes 24) are connected to the tympanic membrane 16 and transmit the acoustic vibrations representing the auditory sound through the middle ear space 26. The middle ear space 26 is vented to the nasopharynx 28 oy the Eustachian tube 30. The ossicles 18 then transmit the vibrations to the inner ear 32, represented by the cochlea 34. The cochlea 34 is a spiral, fluid filled organ which transforms the acoustic vibrations into nerve discharges which are transmitted to the brain (not shown) by the VIIIth (eighth) nerve 36. The nerve discharges are recognized by the brain as the auditory sensation received by the external ear canal 14.

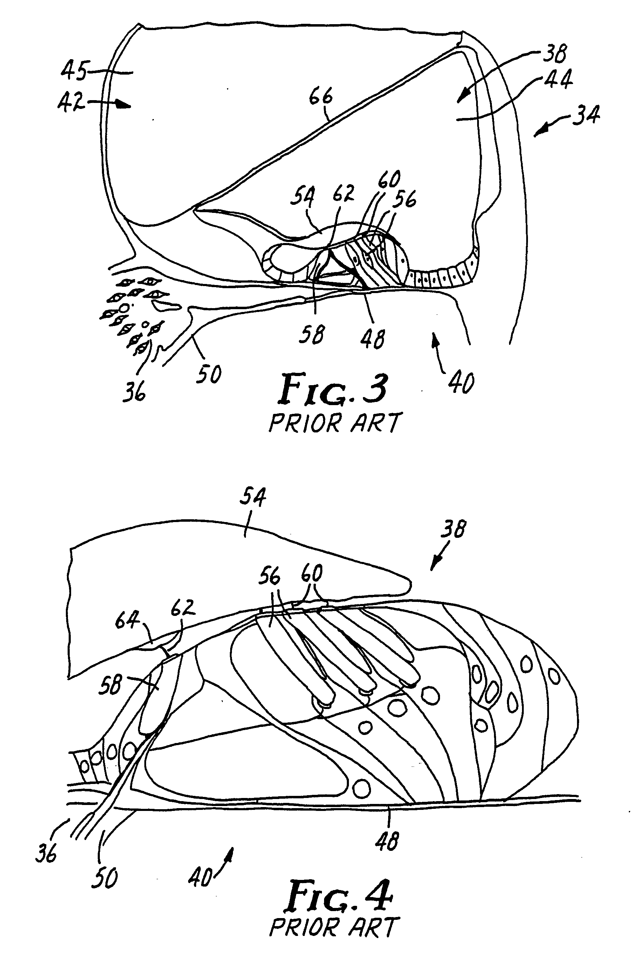

Figure 2 illustrates the anatomy of the cochlea 34 by showing a cross-sectional view. The section of the cochlea 34 represented by the illustration of Figure 2 shows the anatomy at one particular location around the spiral of cochlea 34. Other locations around the spiral would look similar but would be responsible for a different perceived frequency of sensation. The cochlea 34 is known to be place-frequency sensitive, i. e., a place, or position, around the spiral is sensitive only to particular frequencies of sound sensation. It is also known that the spiral of the cochlea 34 is sensitive to increasingly lower frequencies as the position along the spiral moves increasingly toward the apex of the cochlea.

The cross-section of the cochlea 34 is composed of three separate compartments, namely the scala media 38, the scala tympani 40 and the scala vestibuli 42. The scala media 38 is filled with a fluid 44 known as the endolymph. The scala tympani 40 and the scala vestibuli 42 are filled with a fluid 45 known as the perilymph. The outer two compartments, namely the scala tympani 40 and the scala vestibuli 42, communicate with each other at the apex of the cochlea 34. The fluid 44 in the central compartment, namely the scala media 38, does not communicate with the fluid 45 in the two outer compartments, namely the scala tympani 40 and the scala vestibuli 42.

Reterence may now be had to Figure 2 and to Figure 3 which illustrate a portion of the cross-section of the cochlea 34 shown in expanded detail. The scala media 38 and the scala tympani 40 are shown as before. Separating the scala media 38 and the scala tympani 40 is the basilar membrane 48. The osseous spiral lamina 50 is bony in construction and not very flexible. The basilar membrane 48 is constructed of more flexible tissue. A gelatinous flap of tissue known as the tectorial membrane 54 extends into the scala media 38. There are four rows (represented by reference numerals 56 and 58) of hair cells within the scala media 38. Three rows of hair cells are referred to as the outer hair cells 56. The fourth row of hair cells are referred to as the inner row of hair cells 58.

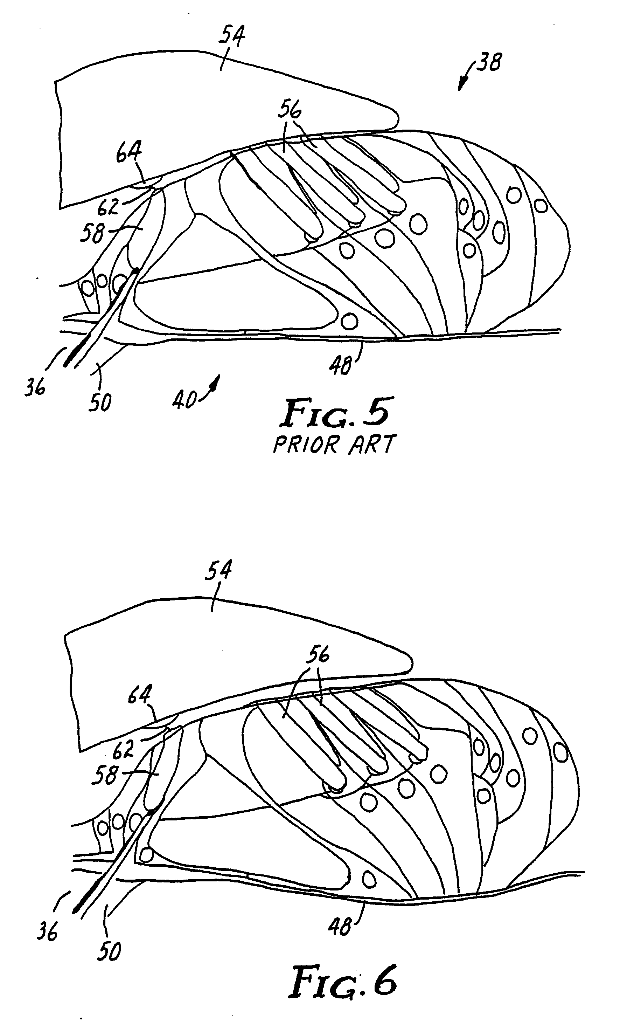

The normal functioning of the cochlea 34 and, hence, the normal functioning of the hearing of an individual, can be explained by reference to Figures 2 and 3, just discussed, and by reference to Figure 4 which illustrates a still further expanded view of a portion of the cochlea 34 showing the scala media 38, the scala tympani 40, the basilar membrane 48, and the relationship of the tectorial membrane 54 to the inner row of hair cells 58 and the outer hair cells 56. The inner row of hair cells 56 is the row of hair cells which is connected to the VIIIth nerve 36 and the row of hair cells which actually transduce auditory input into neural output thus producing auditory sensations. In an individual with normal hearing, the stereocilia 62 of the inner row of hair cells 58 are separated from the tectorial membrane 54 by a small fluid 44 space. The three rows of outer hair cells 56 whose stereocilia 60 are directly attached to the tectorial membrane 54 perform the broad function of transducing the mechanical energy contained in the oscillations of the fluid 44 in the scala media 38 into electrical energy and retransducing that electrical energy back into mechanical energy to provide amplification and to sharpen the tuning of the cochlea. The three rows of outer hair cells 56, by way of their associated stereocilia 60, also maintain a separation of the inner row of hair cells 58, and their associated stereocilia 62, from the tectorial membrane 54. The inner hair cells 56, which are strictly passive, then sense the amplified movement of the basilar membrane 48 and/or tectorial membrane 54 through shearing forces in the fluid 44 which cause deflection of their associated stereocilia 62, depolarization, and release of neuro-transmitter which excites the VIIIth nerve 36.

Figure 5 illustrates a damaged cochlea 34 typical of the damage incurred by an individual having non-Meniere's induced tinnitus. In the cochleae 34 of persons with impaired hearing, particularly those damaged as a result of excessive noise, the damaged and sometimes missing stereocilia 60 of the three rows of outer hair cells 56 may allow the tectorial membrane 54, at least at one location or along a portion of the cochlea 34, to contact the stereocilia 62 of the inner row of hair cells 58 causing deflection of the stereocilia 62, depolarization of the inner hair cells 58 and release of the neuro-transmitter and, thus, excitation of the VIIIth nerve 36 resulting in a sensation of hearing whether or not sound is present, i. e., tinnitus.

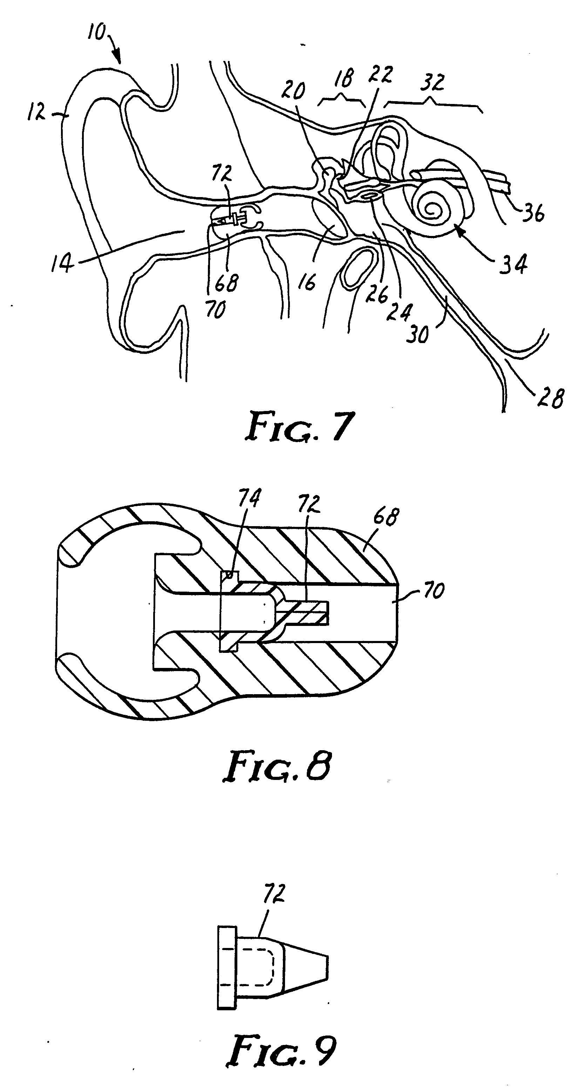

Figure 6 illustrates the damaged cochlea 34 of Figure 5 which has been manipulated according to the method ano apparatus of the present invention thus reducing, i. e., suppressing the tinnitus present. The cure, then, for the prevention or reduction in the sensation of hearing, and to alter other aspects of the auditory sensation, is to separate the tectorial membrane 54 from the stereocilia 62 of the inner row of hair cells 58 where contact has been made. It has been found that displacing the basilar membrane 48, especially a static displacement of the basilar membrane 48 in a direction toward the scala tympani 40 and away from the scala media 38, provides the desired separation of the stereocilia 62 of the inner row of hair cells 58 and results in the reduction or elimination of tinnitus.

In the vast majority of cases involving non-Meniere's tinnitus it is desirable to reposition the basilar membrane 48 statically in a direction away from the scala media 38 and toward the scala tympani 40. As can be seen from Figure 6, repositioning the basilar membrane 48 in this direction causes the tectorial membrane to reestablish its normal position with respect to the stereocilia 62 of the inner row of hair cells 58. With the tectorial membrane 54 repositioned, the tectorial membrane 54, especially Hensen's stripe 64, no longer deflects the stereocilia 62 of the inner row of hair cells 58 (in the absence of actual sound). Since the stereocilia 62 of the inner row of hair cells 58 are no longer chronically contacted by the tectorial membrane 54, the VIII nerve fibers at this location do not discharge excessively in the absence of actual sound and the sensation of tinnitus is completely eliminated. Of course, in an individual in which the exact proper repositioning cannot be achieved, repositioning toward the "normal" would result in a reduction or a suppression of the tinnitus. With the tectorial membrane 54 properly repositioned in its "normal" position, the normal functioning of the inner row of hair cells is reestablished. When vibrations are present in the fluid 44 of the scala media 38, the movement of the tectorial membrane 54 and/or basilar membrane 48 result in the deflection of the stereocilia 62 of the inner row of hair cells 58 located at the position (or location) along the cochlea 34 (and, hence, along the basilar membrane 48) which corresponds to the frequency of the vibrations resulting in the perception of hearing to the sound causing the vibration at that frequency.

Once present, the loss or damage to the outer hair cells 56 and their associated stereocilia 60 is a chronic condition. Similarly, the deflection of the stereocilia 62 of the inner row of hair cells 58 and the resulting tinnitus in this case is a persistent condition, which then suggests a static, or relatively invariant repositioning of the involved structures to acheive a suppression of the tinnitus. The repositioning of the basilar membrane 48 should be functionally invariant, i.e., as stable as needs to be to relieve or suppress the tinnitus. This repositioning preferably needs to vary at less than 20 Hz, since rates above this would result in audible sensations and, although practicle, are considered to be less desirable even though extremely low frequencies have a high auditibility threshhold. Preferably, this repositioning should be functionally invariant such that the separation is maintained between the stereocilia 62 of the inner hair cells 58 and the tectorial membrane 54 for relatively long periods of time, thereby suppressing the tinnitus. The longer the period of time that the structures are repositioned, the greater the duration of the tinnitus suppression. For example, low frequencies of repositioning (e.g. 1 Hz) would be expected to acheive intermittent reduction of the tinnitus however a static, relatively invariant repositioning of the involved structures is preferable to achieve non-intermittent suppression of the tinnitus.

It is generally desirable that the apparatus and method of the present invention be practiced chronically. Although in some cases a residual suppression of the tinnitus has been observed in patients following the removal of the apparatus or the cessation of the method, the nature and efficacy of the residual suppression is generally unknown at this time. There may be instances where the apparatus or method of the present invention could be practiced intermittently. In the periods or times bewteen application of the invention a residual mitigation of the tinnitus may be observed. In general, however, the nature of the present invention involves chronic application to achieve the desired therapeutic benefits.

While it is considered desirable to reposition the basilar membrane 48 statically, such static repositioning is not absolutely required. It is preferable that the repositioning of the basilar membrane 48 be static since the underlying cause of the non-normal positioning of the basilar membran 48 resulting in the tinnitus is relatively static. However, it is to be recognized and understood that other functions, generally not associated with the suppression of tinnitus, may dictate or suggest that the mechanisms associated with the repositioning of the basilar membrane (and, hence, the relationship of the tectorial membrane 54 to the inner row of hair cells) not be completely non-varying. For example, it is contemplated that the present invention could be utilized in conjunction with hearing enhancement mechanisms which may require the variation in the positioning of the basilar membrane. A hearing aid, acoustic or implanted, could be utilized along with the preferable relatively static repositioning of the basilar membrane 48 to create a combination of a relative static repositioning to suppress the tinnitus symptom of the patient's hearing and an amplification or other processing of auditory sensations resulting in variable signals and, hence, variant repositioning of the basilar membrane to achieve the hearing enhancement function of the mechanism.

It is believed that in the typical Meniere's disease patient the fluid pressure in the scala media 38 is higher than normal. This higher than normal pressure results in a deflection of the basilar membrane 48 toward the scala tympani 40 (and also a deflection of Reissner's membrane 66 toward the scala vestibuli 42), a condition known as hydrops. Since in the typical Meniere's disease, the outer row of hair cells 56 would not be damaged, it is thought that the deflection of the basilar membrane 48 could cause the tectorial membrane 54 to be pulled down by the outer row of hair cells 56 to which it would be attached and cause the tectorial membrane 54 to contact the inner row of hair cells 58 along a substantial portion of the cochlea 34 resulting in the typical low frequency roaring tinnitus associated with Meniere's disease. In this situation it may be desirable to reposition the basilar membrane 48 back to its normal position (in this case moving the basilar membrane 48 toward the scala media 38) to relieve the tectorial membrane from contacting the inner row of hair cells. The hydrops condition of Meniere's disease is a relatively static, i. e., relative to auditory sounds, signals or vibrations, condition. A static, relatively invariant, repositioning of the basilar membrane 48 would be preferable. It is preferable that the repositioning does not vary substantially at a rate high enough which would result in audible sensations, otherwise the purpose of suppressing the tinnitus may be defeated. Generally, it is expected that the preferable rate of change for the purpose of repositioning the basilar membrane 48 would be not more than 1 Hertz, still further preferably not more than 20 Hertz.

The Denserts, referred to in the Background have suggested mechanisms to attempt to pump to excess fluid 44 from the scala media 38. In order to do this, they have suggested applying pressure to move the basilar membrane 48 in only one direction, namely toward the scala media 38, and, in order to obtain the pumping that they feel is required, in only a fluctuating pressure which is sometimes superimposed upon a static pressure. Thus, the Denserts' do not achieve the desired result of the present invention because they reposition the basilar membrane 48, if at all, in only the direction toward the scala media 38 and do provide the static repositioning preferred.

It has been found that by modifying the ambient air pressure in the external ear canal 14, the tympanic membrane 16 may be repositioned resulting in a movement of the ossicles 18. The movement of the ossicles 18 results in a change in the pressure of fluid 45 in the cochlea 34. A decrease in the ambient air pressure in the external ear canal 14 causes an outward movement of the stapes 24 and a decrease in the pressure of the fluid 45 in the outer chambers (40 and 42) of the cochlea 34 with a resulting decrease in the pressure of fluid 44 in the scala media 38. These pressure changes are equilibriated by disparate changes in the volumes of fluid 44 and fluid 45 resulting in a relative displacement of the basilar membrane 48. Thus, a decrease in the ambient air pressure in the external ear canal 14 results in a repositioning of the basilar membrane 48 away from the scala media 38 and toward the scala tympani 40. This is the preferred direction of movement of the basilar membrane 48 to suppress tinnitus in patients having noise induced hearing loss, the most common cause of tinnitus.

Thus, it has been found that a change in ambient air pressure in the external ear canal 14 effects the desired repositioning of the basilar membrane 48. By supplying a selected amount of pressure change from ambient (preferably a decrease but possibly, an increase, and if a pressure increase, then preferably statically) the basilar membrane 48 may be repositioned and the tectorial membrane 54 may be properly repositioned in its "normal" position. The normal functioning of the inner row of hair cells is then reestablished. When vibrations are present in the fluid 44 of the scala media 38, the movement of the tectorial membrane 54 and/or basilar membrane 48 result in the deflection of the stereocilia 62 the inner row of hair cells 58 located at the position (or location) along the cochlea 34 which corresponds to the frequency of the vibrations resulting in the perception of hearing to the sound causing the vibration at that frequency.

As discussed before, once present, the loss or damage to the outer hair cells 56 and their associated stereocilia 60 is a chronic condition. Similarly, the deflection of the stereocilia 62 of the inner hair cells 58 and the resulting tinnitus in this case is a persistent condition, which then requires a static, or relatively invariant pressure change in the external ear canal 14 to achieve a suppression of the tinnitus. This repositioning certainly needs to vary at less than 20 Hz, since rates above this would result in audible sensations and defeat the purpose of the tinnitus suppression. Preferably, this pressure change should be relatively invariant such that the separation is maintained between the stereocilia 62 of the inner hair cells 58 and the tectorial membrane 54 for long periods of time, thereby suppressing the tinnitus. The longer the period of time the pressure change (relative to ambient) in the external ear canal 14, the greater the duration of the tinnitus suppression. For example, low frequencies of pressure change (e.g. 1 Hz) would be expected to acheive intermittent reduction of the tinnitus however a static, relatively invariant pressure change in the external ear canal 14 is preferable to achieve non-intermittent suppression of the tinnitus.

While it is considered desirable to supply a static pressure decrease, preferably, or increase to the external ear canal 14, maintaining such a static pressure is not required. It is preferable that the pressure being supplied to the external ear canal 14 be static since the underlying cause of the tinnitus is relatively static. However, it is to be recognized and understood that other functions, generally not associated with the suppression of tinnitus, may dictate or suggest that the mechanisms associated with the pressures being supplied to the external ear canal 14 may not be completely invariant. For example, it is contemplated that the present invention could be utilized in conjunction with hearing enhancement mechanisms which may require variations in the pressures being supplied to the external ear canal 14. A hearing aid could be utilized along with the preferably relatively static pressure being supplied to the external ear canal 14 to create a combination of a relative static pressure to suppress the tinnitus symptom of the patient's hearing and an amplification or other processing of auditory sensations resulting in variant pressures and, hence, variant pressures in the external ear canal 14 to achieve the hearing enhancement function of the mechanism.

Figure 7 illustrates a preferred embodiment of the present invention to suppress or eliminate tinnitus. A plug 68 is shown inserted into the external ear canal 14 of a patient. Preferably the ear plug maintains a good seal with the sides of the external ear canal 14 to result in a substantially hermetic seal, i.e., substantially air tight, of the external ear canal 14 interior of plug 68. Plug 68 has an orifice 70 passing through plug 68 allowing the passage of fluids either into or out of the external ear canal 14 interior of plug 68. It is preferred that the fluid passed, and utilized to modify the pressure in the external ear canal 14 be a gas, in particular air.

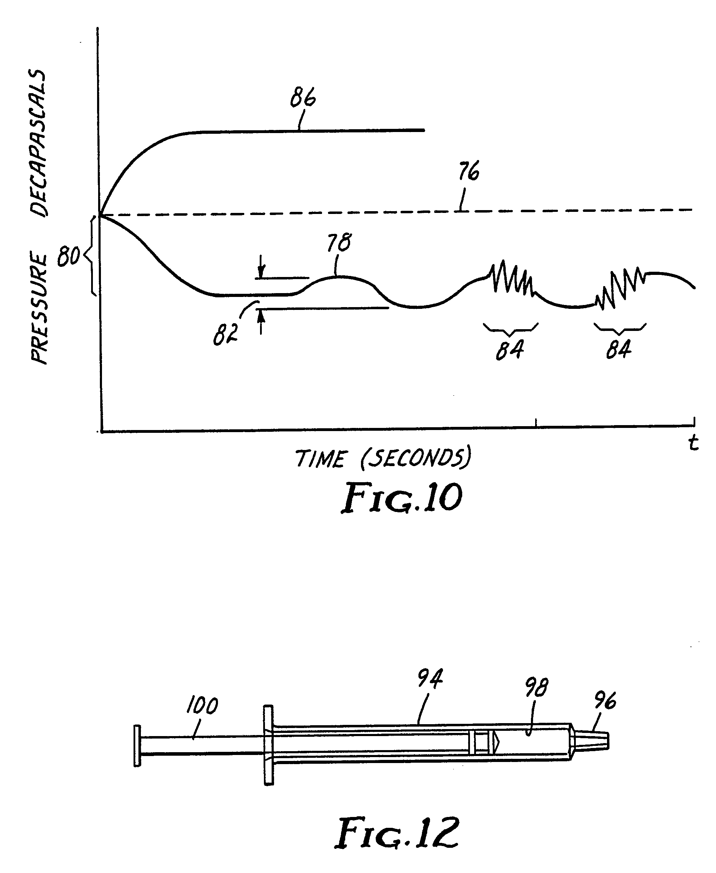

The preferred plug 68 shown in Figure 7 and in more detail in Figure 8 could either be chronically connected to a source of pressure or, preferably, be intermittently, when needed, connected to a source of pressure and the orifice 70 of plug 68 be fitted with a valve 72. Valve 72 will allow the communication of fluids into and/or out of the external ear canal 14 interior of the plug 68 when the plug 68 is coupled to a source of pressure (not shown). When valve 72 is oriented in plug 68 as shown fluids may be passed only out of the interior of the exterior ear canal 14 to reduce the pressure therein relative to ambient. If it is desired to increase the pressure in the external ear canal 14 relative to ambient, the orientation of valve 72 may be reversed. When the plug 68 is disconnected from the source of pressure, valve 72 would maintain the pressure differential within the external ear canal 14. If chronic connection to a source of pressure is desired, then, of course, valve 72 could be eliminated.

An example for plug 68 is shown in Figure 8 and is constructed from a silicone rubber. In early tests with this plug 68 a tip, model 12-2376-9592-4 Universal Soft Ear Tip available from Minnesota Mining and Manufacturing Company of St. Paul, Minnesota from a stethoscope has been utilized with modifications. Plug 68 has orifice 70 running longitidinally through it. A notch 74 is provided along orifice 70 to allow for easy insertion of valve 72. In a preferred embodiment, valve 72, illustrated in Figure 9, is a duckbill valve, such as model VA 3426 available from Vernay Laboratories, Inc., of Yellow Springs, Ohio.

Figure 10 illustrates exemplary pressures, relative to ambient 76, which may achieved in accordance with the present invention. The ordinate of the graph shown in Figure 10 represents time (in seconds) and the abscissa represents pressure (in decapascals). Ambient pressure 76 is shown as a dashed line. In accordance with the present invention, line 78 illustrates a change in pressure, e. g. a decrease in pressure, in the external ear canal 14 interior of plug 68. This pressure decrease is, preferably, maintained relatively constant. Generally this pressure is expected to be maintained over the major portion of the treatment of the tinnitus, although some residual improvement in, i. e., suppression of, tinnitus has been observed following the elimination of the pressure change. Generally, it is expected that the pressure change 80 in the external ear canal 14 interior of the plug 68 which is expected to accomplish the suppression of tinnitus would be in the range of 400-600 decaPascals. Again, a pressure decrease is preferred as shown by line 78, although smaller and greater pressures have been known to accomplish some suppression or be required to achieve complete suppression of the tinnitus. Pressure changes 80 up to 1,000 decaPascals have been utilized. It is expected that, in some individuals, greater pressure cnanges 80 might be tolerable or desirable. At some point, pressure on the tympanic membrane 16 becomes so great that intense pain is experienced militating against its use as a tinnitus suppression mechanism. In order to maintain the preferably static pressure changes illustrated by line 78, it is preferred that the general variation 82 in the pressure change be not more than 10 percent of the total pressure change 80. Generally, since it is desired that the general variation 82 in the pressure not interfere with the auditory sensation, it is preferred that the variation 82 not vary a rate which results in a frequency which is audible. It is preferred that the variation, if any, occur at a rate not more than 1 Hertz, and still preferably not more than 20 Hertz. It is to be recognized that the preferably static pressure change 80 can be utilized in conjunction with other auditory related pressures in the external ear canal 14 associated with improving hearing or ameliorating hearing deficiencies and can be in addition, although not directly related, to the pressure change 80. These additional pressures are represented in the graph of Figure 10 by the changes in pressure indicated by reference numeral 84. Line 86 in the graph of Figure 10 is representative of the situations where it is desirable to have an increased pressure in the external ear canal 14 in order to suppress tinnitus. With the increased pressure represented by line 86, it is preferred that the pressure be static, i. e., relatively invariant.

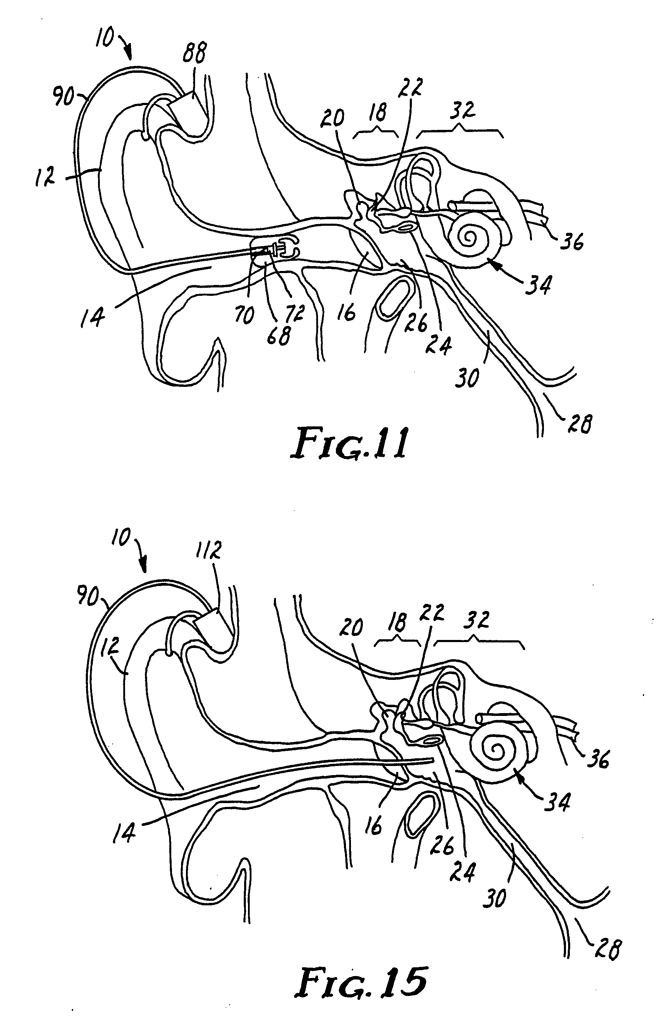

Figure 11 illustrates plug 68 in place in the external ear canal 14 of a patient. The plug, as in Figures 8 and 9, has an orifice 70 with a notch 74. A valve 72 fits within orifice 70 secured by notch 74. With plug 68 in place and without a source of pressure coupled to valve 72, a relatively constant pressure differential (if one had been established) is maintained within the external ear canal 14 interior of plug 68. An example of a source of pressure 88 is shown in Figure 11 coupled to orifice 70 of plug 68 by tube 90. Preferably, the source of pressure 88 is temporarily coupled to plug 68, the appropriate pressure created within the external ear canal 14, and the source of pressure 88 then being disconnected. The source of pressure 88 may then be placed anywhere it is not conspicuous, such as the patient's pocket or a desk drawer. The plug 68, which remains in place in the external ear canal 14, retains the pressure change created, thus maintaining the tinnitus suppression. In another embodiment, the source of pressure may be semi-permanently worn on the body and thus may be secured to the pinna 12 as shown in Figure 11. With the source of pressure 88 being semi-permanently connected to the plug, then the source of pressure 88 would take the place of the valve 72 and since the source of pressure would not ordinarily be disconnected, the valve 72 could be eliminated from plug 68. While in place in either event, the source of pressure 88 would be connected to the orifice 70 of plug 68 and valve 72 would be opened to allow for the passage of fluid, e. g., air. The source of pressure 88 would be operated to achieve the desired pressure differential from ambient in the external ear canal 14 interior of the plug 68. The source of pressure could then be disconnected from plug 68. The near hermetic seal of plug 68 to tne walls of the external ear canal 14 and the closing of valve 72 prior to disconnection would maintain the desired pressure differential. Alternatively, the source of pressure 88 could be chronically coupled by tube 90 to orifice 70 of plug 68. The source of pressure 88 then would variably, or aperiodically, operate to maintain the desired pressure differential within the external ear canal 14 interior of the plug 68. In this alternative construction valve 72 would not be required since the source of pressure would maintain the pressure differential. An example of a source of pressure in this alternative embodiment is an electric pump such as Model 3003 vacuum pump available from ASF Incorporated of Norcross, Georgia. This electric pump is adjustable and may be powered by battery.

Figure 12 illustrates an embodiment for the source of pressure 88. The syringe 94 has a tip 96 which enables the syringe 94 to be disconnectably coupled to orifice 70 of plug 68. Valve 72 allows the passage of fluid only from the interior of the external ear canal 14 outwardly. The fluid in the chamber 98 of the syringe 94 may be varied by moving the plunger 100 in standard typical syringe fashion. To decrease the pressure from ambient in the external ear canal 14 interior of plug 68 (the preferred case) then the plunger 100 of syringe 94 would be positioned at or near the tip 96. The tip 96 of syringe 94 would then be coupled to orifice 70 of plug 68. Plunger 100 of syringe 94 would then be carefully partially extracted from the body of the syringe 94 opening valve 72 and moving fluid from the external ear canal 14 interior of the plug 68 into the chamber 98 of the syringe 94 resulting in a decreased (from ambient) air pressure in the external ear canal 14 interior of plug 68. When the pressure differential stabilizes, valve 72 closes maintaining the pressure differential. The syringe 94 may then be decoupled from plug 68.

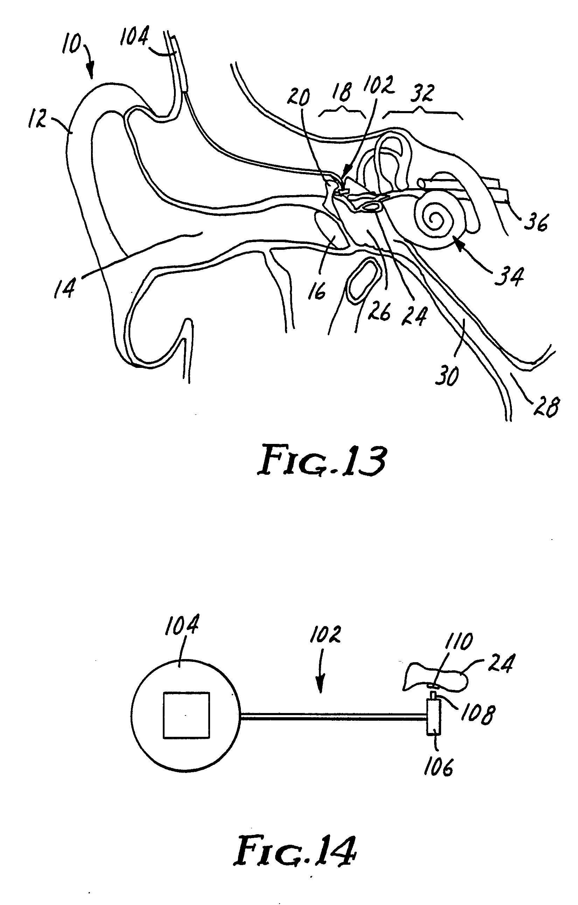

Figure 13 represents an alternative mechanism for achieving the basilar memorane 48 repositioning discussed above. Since a change in position of any element along the path of the ear 10 which changes the pressure of fluid 44 in the scala media 38 will result in a change in the position of the basilar membrane 48, a mechanism which changes the position of the ossicles 18, or any one of the ossicles, namely the malleus 20, the incus 22 or the stapes 24 (refer to Figure 1), can change the position of the basilar membrane 48 to effect a suppression of tinnitus as taught by the present invention. An ossicular displacement device 102 implanted in the middle ear space 26 of a patient is illustrated in Figure 13.

This ossicular displacement device is illustrated in more detail in Figure 14. Control circuitry 104 is selected to mechanically move an ossicle element 106 which is magnetically coupled to one of the ossicles 18, e. g. the stapes 24, by magnets 108 and 110 attached to control circuitry 104 and adhesively attached to the stapes 24, respectively. In order to create the preferred repositioning of the basilar membrane 48 toward the scala tympani 40, the ossicles 18, or one of them, would be repositioned by device 102 in a direction toward the tympanic membrane 16 and away from the inner ear 32.

Alternatively, the objectives of the present invention in suppressing tinnitus could be achieved by supplying a pressure differential, relative to ambient, in the middle ear space 26, rather than the external ear canel 14. The pressure supplied to the middle ear space could be accomplished in two ways.

First, the middle ear space 26 can be isolated from the external ear canal 14 and a pressure differential developed between the middle ear space 26 and the external ear canal 14. Figure 15 illustrates a middle ear pressure device 112 located in the middle ear space 26 of a patient. Instead of directly repositioning the ossicles as does the ossicular displacement device 102, the middle ear pressure device of Figure 15 operates by supplying a pressure differential in middle ear space 26. This mechanism for repositioning the basilar membrane is not preferred, however, since it involves implanting the middle ear pressure device 112 into the middle ear space 26 and since a positive pressure is more difficult to maintain than a negative pressure. The pressure created in the middle ear space 26 would need to be supplemented on a regular basis due to the tendency of the middle ear space 26 to vent to ambient through the Eustachian tube 30. Exemplary devices to be used the middle ear pressure device 112 are those devices suggested for source of pressure 88. Of course, care must be taken in the selection of materials for implantation and in the powering of such a device by a battery. For this reason, it is preferred that middle ear pressure device 112 be similar to source of pressure 88 with tube 90 being extended into the middle ear space 26 thus transferring the pressure created by source of pressure 88 to the middle ear space 26. The preferred insertion spot for tube 90 to enter the middle ear space 26 is through the tympanic membrane 16. The hole created in the tympanic membrane needs to be sealed to the extent to allow the source of pressure 88 to maintain the desired pressure in the middle ear space. As in the external ear canal it is generally preferred that the pressures required be in the 400-600 decaPascals range although pressures as high as 1000 decaPascals could be achieved.

Alternatively, the middle ear space 26 may be vented to the external ear canal 14 with a ventilation tube while a pressure is maintained within the external ear canal 14 by a device. An example of such a pressure device 112 would be the previously described (as in Figure 11) with a ventilation tube 90 extended through the tympanic membrane 16. Thus, the middle ear space 26 would be vented to the external ear canal 14. Any pressure maintained in the external ear canal 14 then would also be maintained in the middle ear space 26. For this reason, the result of the direction of pressure maintained in the middle ear space 26 is vastly different from the same pressure in the middle ear space 26 without venting through the tympanic membrane 16 into the external ear canal 14. Since, the pressure in both places is the same, it is believed that the same pressure effect observed in the case of an external ear canal 14 device applies here. Thus, it is preferred that a pressure decrease with respect to ambient, i. e., a negative pressure, be utilized in order to move the basilar membrane toward the scala tympani 40. Similar pressures would be involved.

Generally a method of the present invention involves the suppressing of tinnitus in a person having a cochlea with a basilar membrane separating the scala media from the scala tympani. The basilar membrane is normally positioned in a first position. The method consists of displacing the basilar membrane from the first position to a second position until the tinnitus is suppressed. In one embodiment the second position, compared with the first position, is located away from the scala media toward the scala tympani. Preferably the displacement is static. As discussed previously, the cochlea 34 has a tectorial membrane 54 and a row of inner hair cells 58 with associated stereocilia 62. Preferably the second position is selected so that the tectorial membrane 54 is separated from the stereocilia 62 associated with the row of inner hair cells 58. Preferably the basilar membrane 54 is displaced to the second position at least for the location along the basilar membrane 48 in the cochlea 34 which corresponds to the location along the basilar membrane 48 in the cochlea 34 causing the tinnitus, generally the location along the basilar membrane 48 which corresponds to the perceived frequency of the tinnitus.

The method of suppressing tinnitus in an auditory system having an external ear canal according to the present invention also utilizes these steps. A substantially hermetic seal is provided to the external ear canal 14 to allow a pressure applied to the external ear canal 14 to be maintained. A plurality of static pressures, having differing values, is then applied to the external ear canal 14. The value or values of the static pressures which suppress the tinnitus are determined. At least one of the values of the static pressures which suppress the tinnitus is maintained for therapeutic benefit achieved. Thus, the tinnitus suffered by the patient is suppressed. Preferably the values of the plurality of static pressures are negative with respect to ambient, i. e., a partial vacuum is created in the external ear canal 14. Preferably the seal will sustain a pressure of at least approximately 1,000 decaPascals. Preferably the determining step is done empirically, i. e., a variety of pressures are utilized and the one or ones which provide the therapeutic benefit are selected. Preferably the pressure is maintained long enough for therapeutic benefit to occur and then ceased. Still further preferably the pressure is maintained chronically, i. e., as long as the therapeutic benefit is desired by the patient.

Thus, it can be seen that there has been shown and described a novel apparatus and method for suppressing tinnitus. It is to be recognized and understood, however, that various changes, modifications and substitutions in the form and the details of the present invention may be made by those skilled in the art without departing from the scope of the invention as defined by the following claims.

## Claims

1. An apparatus for suppressing tinnitus in an auditory system having a cochlea with a basilar membrane separating the scala media from the scala tympani, said basilar membrane being normally positioned in a first position, said apparatus comprising a device displacing said basilar membrane from said first position to a second position suppressing said tinnitus.

2. An apparatus for suppressing tinnitus as in claim 1 wherein said second position, compared with said first position, is away from said scala media and toward said scala tympani.

3. An apparatus for suppressing tinnitus as in claim 1 wherein said displacement is a static displacement.

4. An apparatus for suppressing tinnitus having an auditory system with a tympanic membrane, a middle ear and an external ear canal, said tympanic membrane being normally positioned in a first position, said apparatus comprising a device adapted to act upon said tympanic membrane displacing said tympanic membrane from said first position to a second position suppressing said tinnitus.

5. An apparatus for suppressing tinnitus as in claim 4 wherein said second position, compared with said first position, is away from said middle ear toward said external ear canal.

6. An apparatus for suppressing tinnitus as in claim 5 wherein said displacement is static.

7. An apparatus for suppressing tinnitus having an auditory system with an ossicular chain in a middle ear and an external ear canal, said ossicular chain being normally positioned in a first position, said apparatus comprising a device adapted to act upon said ossicular chain displacing said ossicular chain from said first position to a second position suppressing said tinnitus.

8. An apparatus for suppressing tinnitus as in claim 7 wherein said second position, compared with said first position, is away from said middle ear toward said external ear canal.

9. An apparatus for suppressing tinnitus as in claim 7 wherein said displacement is static.

10. An apparatus for suppressing tinnitus having an auditory system with an ossicular chain in a middle ear and an external ear canal, said ossicular chain being normally positioned in a first position, said apparatus comprising a device adapted to act upon said ossicular chain displacing said ossicular chain from said first position to a second position by creating a negative, relative to ambient, pressure differential in said middle ear suppressing said tinnitus.

11. An apparatus for suppressing tinnitus as in claim 10 wherein said second position, compared with said first position, is away from said middle ear toward said external ear canal.

12. An apparatus for suppressing tinnitus as in claim 11 wherein said pressure differential is static.

13. An apparatus for suppressing tinnitus in an auditory system having a cochlea with a basilar membrane separating the scala media from the scala tympani, said basilar membrane normally physically positioned in a first position, said apparatus comprising:
mounting means adapted to be utilized in conjunction with said auditory system for mounting said apparatus to said person; and
displacement means coupled to said mounting means and adapted to act upon said basilar membrane for displacing said basilar membrane from said first position to a second position suppressing said tinnitus.

14. An apparatus for suppressing tinnitus as in claim 13 wherein said second position, relative to said first position, is away from scala media and toward said scala tympani.

15. An apparatus for suppressing tinnitus as in claim 13 wherein said displacement is static.

16. An apparatus for suppressing tinnitus in a person having an external ear canal, comprising:
sealing means adapted to be coupled to said external ear canal for providing a substantially hermetic seal, relative to ambient, for said external ear canal; and
pressure means adapted to be coupled to said external ear canal for providing a pressure, relative to ambient, in said external ear canal suppressing said tinnitus.

17. An apparatus for suppressing tinnitus as in claim 16 wherein said pressure, relative to ambient, is negative.

18. An apparatus for suppressing tinnitus as in claim 16 wherein said pressure is static.

19. An apparatus for suppressing tinnitus as in claim 17 which further comprises maintenance means coupled to said sealing means for maintaining said pressure in said external ear canal with said apparatus in place.

20. A method of suppressing tinnitus in an auditory system having an external ear canal, comprising the steps of:

providing a substantially hermetic seal to said external ear canal;

applying a plurality of static pressures, having differing values, to said external ear canal;

determining which value or values of said static pressures suppresses said tinnitus; and

maintaining at least one of said values of said static pressures for therapeutic benefit;

whereby said tinnitus is suppressed.

21. A method of suppressing tinnitus as in claim 20 wherein said values of said plurality of static pressures are negative with respect to ambient.

22. A method of suppressing tinnitus as in claim 21 wherein said providing step provides a seal that will sustain a pressure of up to approximately ± 1,000 decaPascals.

23. A tinnitus suppression device adapted to be utilized in conjunction with a person having an auditory system with an external ear canal, said device comprising:

an ear plug adapted to be placed in said external ear canal of said person, said ear plug having an orifice allowing communication of fluids to and from said external ear canal with said ear plug in place within said external ear canal; and

pressure means coupled to said orifice of said ear plug for providing a selected pressure to be applied to said external ear canal with said ear plug in place;

whereby said device maintains said selected pressure within said external ear canal suppressing said tinnitus.

24. A tinnitus suppression device as in claim 23 further comprising valve means coupled to said ear plug for selectively allowing fluids to be transferred into or out of said external ear canal through said orifice, said pressure means being coupled to said valve means opening said valve means allowing said pressure means to transfer fluids into or from said external canal, said pressure means being disconnectable from said valve means, said valve means maintaining said selected pressure within said external ear canal when said pressure means is disconnected.

25. A tinnitus suppression device adapted to be utilized in conjunction with a person having an auditory system with an external ear canal, said device comprising:

sealing means, sized and shaped for easy insertion into said external ear canal of said person, said sealing means having an orifice allowing for the communication of fluids in and out of said external ear canal with said device in place within said external ear canal for sealing said external ear canal from ambient; and

pressure means coupled to said orifice of said sealing means for providing a selected pressure to be applied to said external ear canal with said device in place;

whereby said device maintains said selected pressure within said external ear canal suppressing said tinnitus.

26. A tinnitus suppression device as in claim 25 wherein said sealing means provides a substantially hermetic seal for said external ear canal.

27. A tinnitus suppression device as in claim 25 wherein said fluid is air.

28. A tinnitus suppression device as in claim 27 wherein said valve only allows fluids to be transferred out of said external ear canal.

**FIG.1**
PRIOR ART

**FIG.2**
PRIOR ART

**FIG. 3**
PRIOR ART

**FIG. 4**
PRIOR ART

FIG. 5
PRIOR ART

FIG. 6

FIG. 7

FIG. 8

FIG. 9

FIG.10

FIG.12

FIG.11

FIG.15

FIG.13

FIG.14

European Patent Office

**PARTIAL EUROPEAN SEARCH REPORT**
which under Rule 45 of the European Patent Convention
shall be considered, for the purposes of subsequent
proceedings, as the European search report

Application number

EP 89311877.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁵) |
|---|---|---|---|
| X | US - A - 2 014 009 (A.T. VANCE)<br>* claim 1; figure 1; page 2, left-hand column, line 64 - right-hand column, line 2 * | 1-4 | A61F11/00 |
| A | | 7,10,13,<br>16,23,25 | |
| A | DE - C - 493 956 (H. HUCKSCHLAG)<br>* claim; figure 1 * | 1-4 | |
| A | FR - A - 2 605 516 (J.P. ROSSI et al.)<br>* claim 1; figure 1 * | 1-4,7,10,<br>13,16,23,<br>25 | |
| D,A | EP - A - 0 266 474 (B. DENSERT et al.)<br>* claim 1 * | 1 | |
| D,A | WO - A - 83 02556 (B. DENSERT et al.)<br>* claim 1 * | 1 | |

TECHNICAL FIELDS
SEARCHED (Int. Cl.⁴)

A61F

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:
Claims searched incompletely: 1-19,23-28
Claims not searched: 20-22: Art. 52(4) method for treatment
Reason for the limitation of the search: of the human or animal body by therapy

| Place of search | Date of completion of the search | Examiner |
|---|---|---|
| BERLIN | 22-02-1990 | P.K. KANAL |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03 82